# EUROPEAN PATENT APPLICATION

(11) **EP 1 757 320 A1**
(43) Date of publication of application: **28.02.2007**
(21) Application number: 05405495.2
(22) Date of filing: 25.08.2005
(51) Int. Cl.: A61M 5/178, B65B 3/00, A61J 1/00, A61M 5/00, B65B 1/04

(54) **Administration device**

(71) Applicant: Eiholzer, Urs, 8006 Zürich (CH)
(72) Inventor: Eiholzer, PD Dr. med. Urs, 8006 Zürich (CH); Neukomm, Prof. Dr. Peter A., 5430 Wettingen (CH)
(74) Representative: Heinen, Detlef

(57) **Abstract**

A device for the administration of an essentially liquid compound or mixture of compounds (100,110), in particular for the administration of an essentially liquid medicament, comprises an administration pen (AP) for the administration of an individual metered dose of the liquid compound or mixture of compounds (100,110). The device further comprises a docking station (DS) to which the administration pen (AP) is to be docked (PD1). The docking station (DS) comprises a dosing station (DOS) for automatically metering the individual dose to be administered and a transfer station (TS) for automatically transferring the individual metered dose to the administration pen (AP).

## Description

The present invention deals with a device for the administration of an essentially liquid compound or mixture of compounds according to the preamble of independent claim 1 and to a cartridge for insertion into a casing of the docking station of the afore-mentioned device.

For the administration of medicaments or hormones, e.g. human growth hormone (STH), shot pens are frequently used in order to assist patients in self-administering the hormone, thus making life much more convenient for these patients. In this respect, the term "essentially liquid" is intended to not only comprise pure liquids or mixtures of pure liquids but also to comprise suspensions, emulsions, etc. which can be administered using shot pens or syringes.

Only a few manufacturers are able to provide STH having sufficient long-term stability in an essentially liquid form, so that in general the STH can be directly administered as it is with the aid of a shot pen. The disadvantage of the liquid form is, that liquid STH requires continuous cooling of the liquid STH starting at the manufacturer and being maintained until the liquid STH is administered. The liquid STH is typically provided in single dose or multi-dose vials, which are to be inserted into the administration pen (shot pen). Due to the requirement for continuous cooling of the liquid STH, the essential effort involved in the administration of each single dose often results in low motivation of the patient, or even worse in poor compliance with the prescribed administration scheme. Particularly at night the administration may turn out to be especially troublesome (since the STH liquid has to be stored in the fridge), all the more when considering that some of the patients are children. In addition, since the administration pens should preferably be light-weight and of small dimensions, it is not possible to for the pen to include an electronic control or a cooling unit.

Other manufacturers provide STH having sufficient long-term stability in the form of a lyophilised powder. While the lyophilised powder exhibits a good long-term stability, it suffers from the disadvantage that it has to be mixed with a liquid solvent to form a liquid solution that can be administered. Usually, a plurality of doses of lyophilised powder are provided in a container and are mixed with the liquid solvent at a single time. In a known administration system, this mixing operation is performed using a separate mixing device. However, the mixing must be performed manually and appears complicated and prone to defects to the patient. Once mixed with the liquid solvent, the container containing the liquid solution to be administered is inserted into the administration pen. The pen must then be stored in the refrigerator so as to maintain the stability of the STH-solution over the period of the multiple administrations. Accordingly, each time the patient needs to administer a dose the patient has to go the fridge and get the pen. This is particularly troublesome at night, in particular when considering that some of the patients are children. Also, the cold temperature of the pen stored in the fridge is inconvenient to many children. This may again result in low motivation of the patient, or even worse in poor compliance with the prescribed administration scheme.

Since both motivation and compliance play an essential role for the success of a treatment, and also in view that a treatment comprising administration of STH is very expensive, it is an object of the invention to devise an administration device which overcomes the disadvantages of the prior art administration devices discussed above and which is easy to use for the patients, thus maintaining the patients' motivation and compliance at a high level.

This object is achieved with an administration device as it is characterized by the features of the independent claim directed to such device. Advantageous embodiments of the device are the subject of the respective dependent claims.

In particular, the device according to the invention comprises a docking station to which the administration pen is to be docked. The docking station comprises a dosing station for automatically metering the individual dose to be administered, and a transfer station for automatically transferring the individual metered dose to the administration pen. Depending on whether the essentially liquid compound or mixture of compounds needs to be cooled or not, the docking station may further comprise a cooling unit for automatically storing the liquid compound or mixture of compounds to be metered, e.g. the growth hormone STH, at a temperature within a predetermined temperature range. The only actions that the user has to perform in order to load the administration pen is to dock the pen to the docking station and wait a short instance until the pen is automatically loaded with the required dose to be administered. The pen loaded with the dose to be administered can then be taken off of the docking station and the shot can then be performed. This is very convenient for the user.

Of course, in case a mixing operation must be performed the docking station according to a further embodiment may comprise a mixing station for automatically mixing a first and a second compound so as to form the essentially liquid mixture of compounds to be metered. For example, the STH powder described above may be mixed with the solvent to form a liquid STH solution prior to metering the required dose. Since the mixing operation is also performed automatically the comfort for the patient is maintained at a constantly high level. It goes without saying that the mixed solution can be placed in the cooling station automatically.

In accordance with a further embodiment of the device according to the invention, the docking station further comprises a syringe reservoir for providing one or more unused syringes. A syringe in the sense of this invention is not a syringe in the conventional sense (stand alone system) but denotes a single use reservoir comprising a piston head and having an outlet with or without a needle. The piston head of the syringe can be actuated after having inserted the syringe into the administration pen so as to move the piston head of the syringe in order to press the liquid STH through the outlet of the syringe (with or without needle). Accordingly, the docking station comprises transport means for automatically transporting an unused syringe from the syringe reservoir to the dosing station for automatically filling the syringe with the metered dose of the compound or mixture of compounds. Once the syringe has been filled with the metered dose, the transfer station of this embodiment of the docking station, which is adapted to automatically insert the filled syringe into the administration pen, transfers the syringe into the pen. Again, all operations are performed automatically so that the comfort for the patient is maintained at a high level.

In a further embodiment of the device according to the invention, the administration pen comprises an actuator means to be actuated by the user. The actuator means comprises a tensioning means and a release means. The docking station of this embodiment comprises a loading means for acting upon the tensioning means such that the actuator means of the pen rests in a tensioned state. This embodiment is advantageous in that even the pen is loaded automatically so that the only operation to be performed by the patient is to take the pen off of the docking station and to actuate the release means in order to start administration of the dose. This is extremely convenient for the patient.

According to a further embodiment of the device according to the invention, the docking station may further comprise a collector station for collecting used syringes to which the administration pen can be docked after administration. The collector station comprises means for actuating on the administration pen upon docking of the administration pen in a manner such that the used syringe is automatically transferred from the administration pen to the collector station. Accordingly, after the administration of the dose the patient only has to dock the administration pen to the collector station, and the used syringe is then automatically transferred from the administration pen to the collector station. The used syringes can thus be collected and recycled. Optionally, the collector station can be designed such that once a used syringe has been transferred from the administration pen to the collector station it is practically impossible to get a used syringe out of the collector station, so that abuse of used syringes is prevented.

In accordance with a specific embodiment of the device according to the invention, the administration pen may comprise a lock means for locking the syringe within administration pen prior to insertion of the administration pen into the means for actuating on the administration pen upon docking it to the docking station. Upon docking the administration pen to the docking station the lock means of the administration pen are actuated by the means for actuating on the administration pen so as to unlock the syringe from the pen, so that it may fall down into the collector station. This is a comparatively inexpensive practical embodiment of the way, how the syringe can be locked and unlocked within and from the administration pen.

In accordance with still a further embodiment of the invention, the docking station or the pen comprises electronic means for the control and/or surveillance of the administration of the liquid compounds or mixture of compounds. For example, in case there is an amount of liquid STH left in the multi-dose containment which is smaller than the next dose to be administered, the electronic means may be adapted to calculate the additional amount of liquid STH needed from the next multi-dose containment. Since STH is very expensive, it is thus possible to completely empty the STH provided in a multi-dose containment and then to add to the syringe the amount necessary to complete the required dose of STH to be administered from the next multi-dose containment. Also, the electronic means may be suitable to store the amounts of STH administered and the time of administration so as to allow surveillance by the physician of the compliance of a patient with a prescribed administration regime. For example, the data so stored can be transferred to the physician wireless or through a memory stick. Of course, additional electronic functions can be incorporated into the pen, if desired, e.g. an alarm indicating to the patient that the time for the administration has come, etc..

A further aspect of the present invention is related to a cartridge for insertion into a casing of a docking station of a device according to the invention. The cartridge comprises a first containment containing a plurality of doses of an essentially liquid compound or mixture of compounds to be administered, or containing a component (e.g. the STH-powder) of an essentially liquid mixture of compounds (e.g. a liquid STH-solution) to be administered in individual doses to a user. The cartridge further comprises a second containment being empty (in case the first cartridge contains the essentially liquid compound to be administered) or containing a second compound (e.g. a liquid solvent) to be mixed with the first compound so as to form the essentially liquid mixture of compounds (e.g. a liquid STH-solution) to be administered. Such cartridge is advantageous in that it simplifies the handling for the patient: The patient simply has to replace the cartridge in the casing of the aforedescribed docking station and then the administration device is again ready for use. The used cartridge can then be returned for recycling purposes. In a specific embodiment the cartridge may further comprises a containment for collecting used syringes which can be returned together with the cartridge.

Further advantageous aspects of the administration device of the present invention or of the cartridge, respectively, will become apparent from the following detailed description of an embodiment of the administration device (or of parts thereof) and the cartridge with the aid of the drawings in which:
- Fig. 1: shows a top view of a docking station according to the instant invention with an inserted cartridge (conveyor and cooling unit not shown)
- Fig. 2: shows a perspective view of a new unused cartridge to be inserted into the docking station of Fig. 1
- Fig. 3: shows a perspective view of a cartridge to be returned after use
- Fig. 4: shows in a cross-sectional view a mixing station of the docking station of Fig. 1
- Fig. 5: shows a cross-sectional top view of the docking station with a conveyor being arranged in the position in which the mixing operation is performed
- Fig. 6: shows a detailed cross-sectional side view of the mixing station shown in Fig. 4 prior to starting the mixing operation
- Fig. 7: shows a cross-sectional view of the mixing station of Fig. 4 during the mixing operation
- Fig. 8: shows a top view of the docking station with the conveyor being arranged in the drop position
- Fig. 9: shows a detailed cross-sectional view of the docking station with the conveyor being arranged in the drop position
- Fig. 10: shows a cross-sectional view of the cooling unit of the embodiment of the docking station according to Fig. 1
- Fig. 11: shows a top view of the cooling unit in the docking station of Fig. 1
- Fig. 12: shows a top view of docking station of Fig. 1 indicating the position in which a push rod for loading the administration pen is arranged
- Fig. 13: shows a cross-sectional view showing the push rod indicated in Fig. 12
- Fig. 14: shows the parts of an embodiment of an administration pen according to the invention, unassembled
- Fig. 15: shows the embodiment of the administration pen of Fig. 14, assembled, above a pen docking element of a pen docking element of the pen docking station according to the invention,
- Fig. 16: shows a cross-sectional view of a detail at the upper end of administration pen
- Fig. 17: shows a cross-sectional top view of the docking station with the conveyor being arranged in the pick-up position for picking up a new syringe, with the arms of the clamp in the open position
- Fig. 18: shows a detailed cross-sectional side view of the docking station with the conveyor being arranged in the pick-up position of Fig. 17
- Fig. 19: shows a cross-sectional top view of the docking station in the position of Fig. 17, but with the arms of the clamp in the closed position
- Fig. 20: shows a detailed cross-sectional side view of the docking station with the conveyor being arranged as shown in Fig. 19
- Fig. 21: shows a cross-sectional top view of the docking station in the dosing position, with the needle of the syringe being in communication with the containment for automatically filling a metered dose into a syringe
- Fig. 22: shows a cross-sectional side view of the dosing station of the docking station as shown in Fig. 21
- Fig. 23: shows a cross-sectional top view of the docking station with the conveyor still being in the dosing position, but with the syringe having been lifted
- Fig. 24: shows a cross-sectional side view of the docking station with the conveyor being in the position of Fig. 23
- Fig. 25: shows a cross-sectional top view of the docking station with the conveyor carrying the syringe being arranged in the transfer position for insertion of the syringe into the pen
- Fig. 26: shows a cross-sectional side view of the docking station showing the transfer station with the conveyor being in the position of Fig. 25
- Fig. 27: shows a cross-sectional top view of the docking station with the conveyor being in the transfer position, with the syringe having been inserted into the pen (clamps widely open)
- Fig. 28: shows a cross-sectional side view of the docking station according to Fig. 27, with the syringe having been transferred to the pen
- Fig. 29: shows an enlarged view of a filled syringe
- Fig. 30: shows an embodiment of the administration pen fully assembled, with loaded spring and syringe inserted, ready for administration
- Fig. 31: shows the pen docking station for automatically releasing the syringe from the pen, and
- Fig. 32: shows a three-dimensional representation of the docking station schematically showing some elements of the docking station.

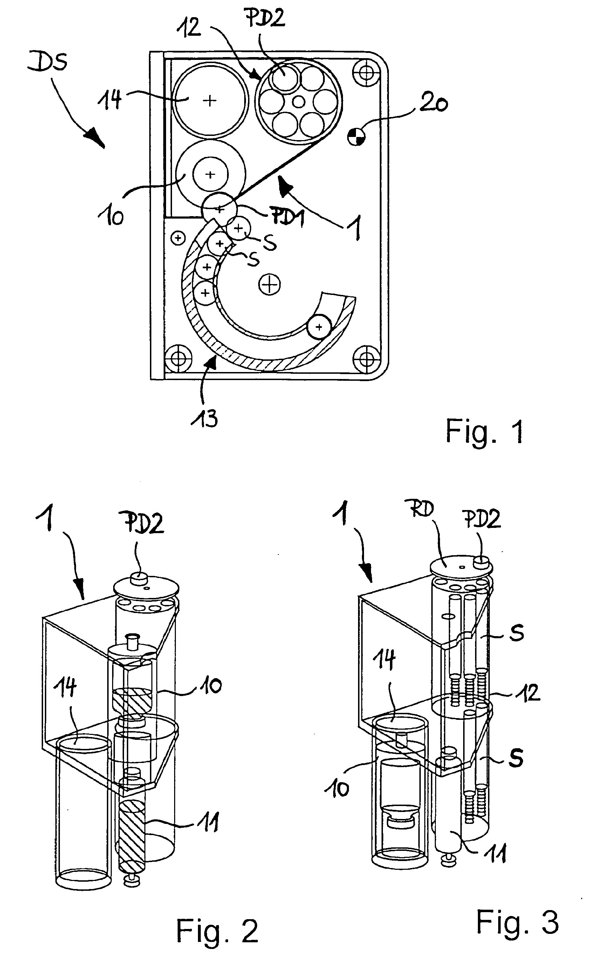

In **Fig. 1** there is shown a top view of the docking station DS of an embodiment of the administration device according to the instant invention. A cartridge 1, which is shown in **Fig. 2** (unused) and **Fig. 3** (used) in a perspective view, has been inserted into the casing of the docking station DS.

Cartridge 1 comprises a first (upper) containment 10 containing, by way of example, an STH-powder to be mixed with a liquid solvent contained in a second (lower) containment 11 so as to form a liquid solution to be administered. Cartridge 1 further comprises a collector drum 12 having axial bores therein for insertion of used syringes S. Fig. 2 shows an unused cartridge 1, and therefore no used syringes S are contained in the collector drum 12. Also, after the mixing of the STH-powder and the liquid solution has been performed, the first containment 10 is moved to a drop position, where it is dropped to a lower level through a corresponding opening 14 provided in the base of cartridge 1 (see Fig. 3). Also, in Fig. 3 a plurality of used syringes are shown in collector drum 12. One way how these operations (mixing of STH-powder with liquid solution, moving containment 10 to the drop position, etc.) can be performed, will be explained in more detail below.

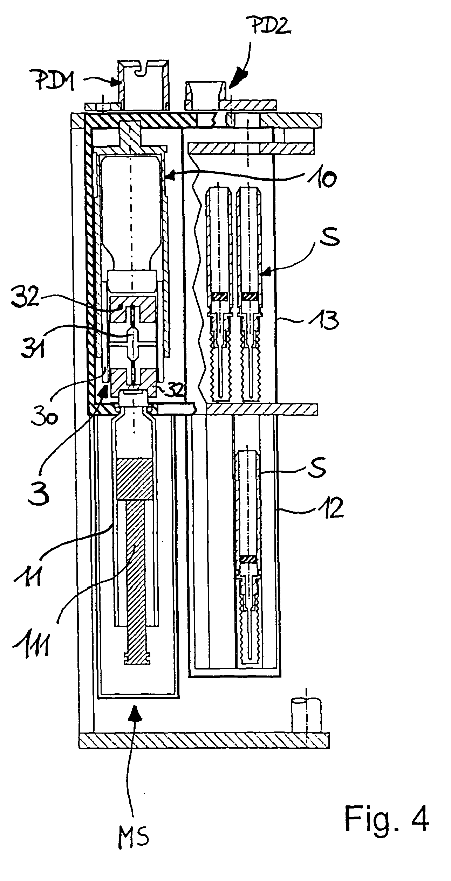

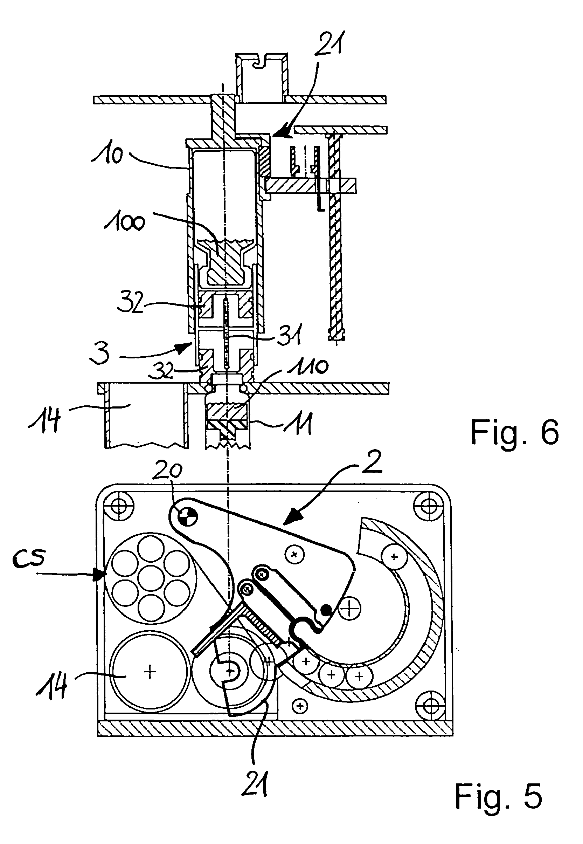

Returning to Fig. 1, docking station DS further comprises a reservoir drum 13 for new unused syringes S, which can be grasped and moved to the dosing station for automatic metering of a dose of the liquid STH-solution into the syringe S. The metering operation will be explained in further detail later. Also, docking station DS comprises two pen docking elements PD1 and PD2 (see also Fig. 4) which are intended for docking the administration pen to docking station DS. When the pen has been engaged with first docking element PD1 the pen can be automatically loaded and a syringe S filled with a metered does of the STH-solution can be automatically inserted into the pen, as will be explained in more detail later. When the pen is engaged with second pen docking element PD2 after administration of a dose, the used syringe S can be automatically released from the pen and falls down into collector drum 12. This will also be explained in more detail later. Axle 20 shown in Fig. 1 represents an axle about which a conveyor 2 (see Fig. 5) can rotate.

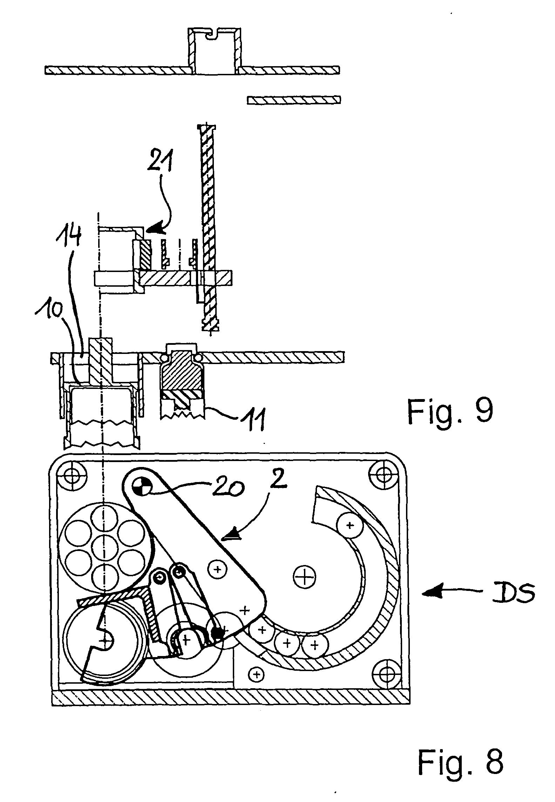

With the aid of Figs. 4-7 the mixing operation will now be explained in more detail, while Fig. 8 and Fig. 9 show the move-and-drop operation of the first containment 10 after the mixing operation has been performed. Accordingly, Fig. 4 shows a cross-sectional view through the docking station DS and in particular shows the mixing station MS. At the mixing station, the first (upper) containment 10 and the second (lower) containment 11 are aligned relative to one another. A connector 3 comprising a connector housing 30, a connecting needle 31 fixedly arranged within connector housing 30, and two sealing plugs 32 movably arranged within connector housing 30 are shown. Also, unused syringes S which are provided in reservoir drum 13 (see Fig. 1) are shown in Fig. 4 as well as used syringes S in collector drum 12.

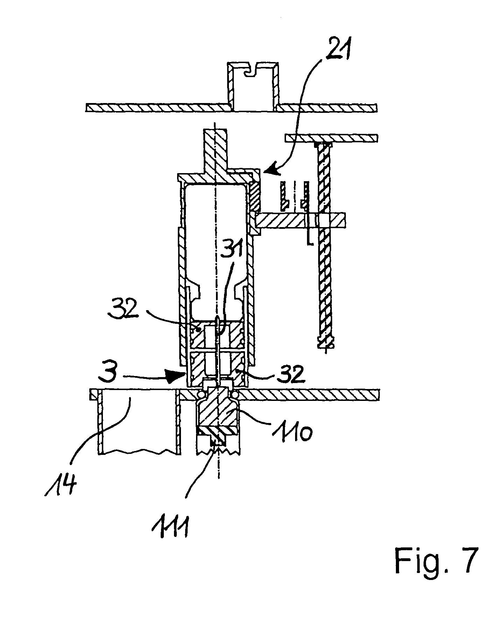

**Fig. 5** shows the conveyor 2 arranged in the position where the mixing operation is performed. Without describing conveyor 2 in more detail it is to be mentioned that it comprises a carrier 21 for carrying and moving upper containment 10. At the beginning, upper containment 10 and lower containment 11 are not connected via connector 3, as is shown in **Fig. 6**. Conveyor 2 then moves downwardly. Since lower containment 11 is fixedly arranged, connecting needle 31 pierces through sealing plugs 32 and thus establishes a fluid communication between the STH powder 100 contained in upper containment 10 and the liquid solvent 110 contained in lower containment 11 (see **Fig. 7**). Once the communication between upper containment 10 and lower containment 11 has been established, a piston 111 (see also Fig. 4) arranged in lower containment 11 is moved upwardly thus pressing the liquid solvent 110 through connecting needle 31 into upper containment 10 and allowing the STH powder 100 to mix with solvent 110. Piston 111 is then again be moved downwardly thus sucking the STH solution (STH powder solved in the liquid solvent) through connecting needle 31 back into lower containment 11. In order to further improve the mixing operation, again an upward stroke of piston 111 can be performed thus pressing the solution again into upper containment 10. Subsequently, piston 111 is again moved downwardly thus sucking the solution back to lower containment 11. If deemed appropriate, additional upward and downward strokes of piston 111 can be performed.

At the end of the mixing operation the STH solution to be administered is contained in lower containment 11. Conveyor 2 is then pivoted about axle 20 (see **Fig. 8**) thus moving the empty upper containment 10 towards opening 14 into the drop position. At the time this drop position is reached, carrier 21 is disengaged from upper containment 10 causing empty upper containment 10 to fall down through opening 14 (see **Fig. 9**), since it is no longer needed.

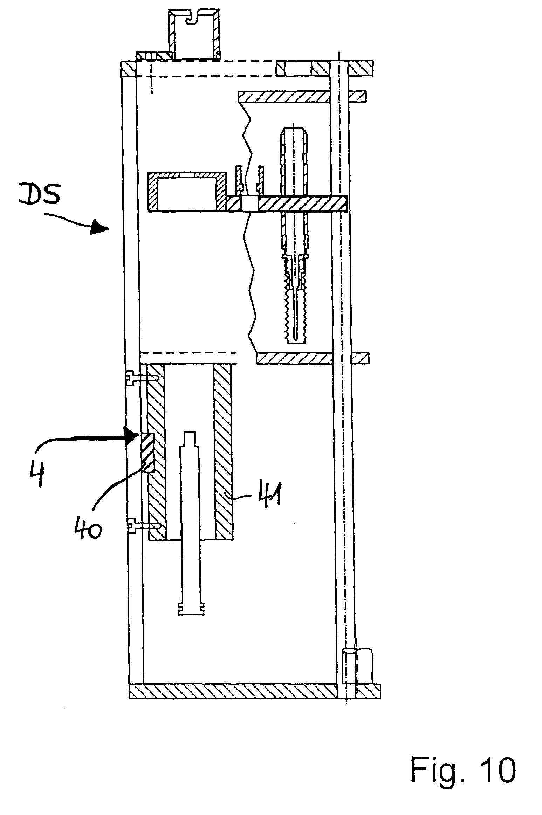

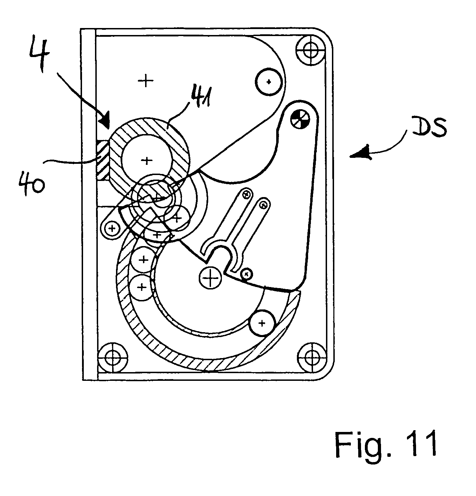

As already discussed further above, the liquid STH solution, once prepared, is preferably to be stored at lower temperatures so as to improve stability of the STH-solution. On the other hand, the patient's comfort is to be maintained at a high level so as to avoid that during the night the patient has to go to the fridge in order to get the STH-solution. For that reason, docking station DS comprises a cooling unit 4 comprising essentially a Peltier-element 40 connected to a highly heat-conductive and heat radiating casing 41 that surrounds lower containment 11. Heat-conductive/heat radiating casing 41 is conventional and is therefore not shown in detail: For example, it may comprise a (cold) inner heat-conductive wall for cooling lower containment 11 containing the STH-solution, an outer heat-conductive/heat radiating wall for transporting heat to the outside, and an insulating element arranged between these two walls for insulating the (cold) inner wall from the (warm) outer wall; condensate that may eventually occur is transported to the outer (warm) wall and evaporates there. The arrangement of cooling unit 4 comprising Peltier-element 40 and casing 41 within docking station DS is shown in **Fig. 10** and **Fig. 11**, respectively, however, lower containment 11 is not represented in these figures.

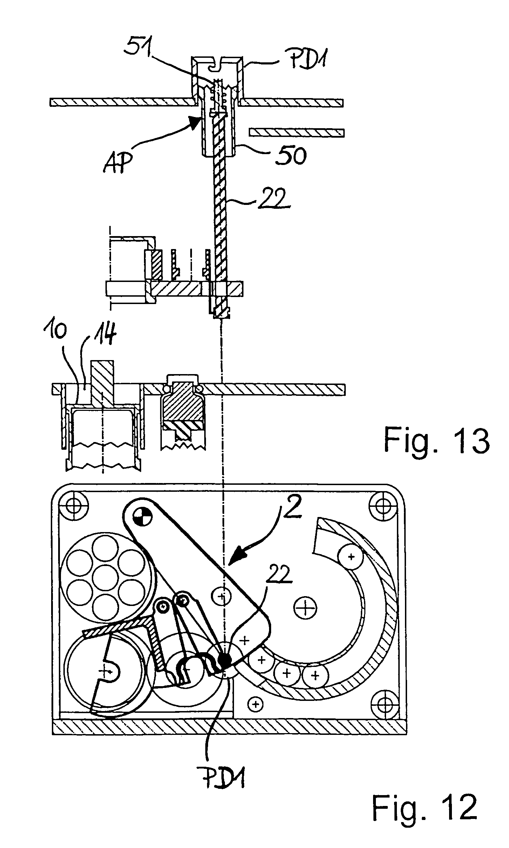

When the mixing operation has been performed, the administration pen can be automatically loaded. The term "loaded" in this respect is to be understood in the sense such that the pen is prepared to a state in which it is later on - during administration of a dose - simply to be released by the user. This will be explained with the aid of Fig. 12 and Fig. 13.

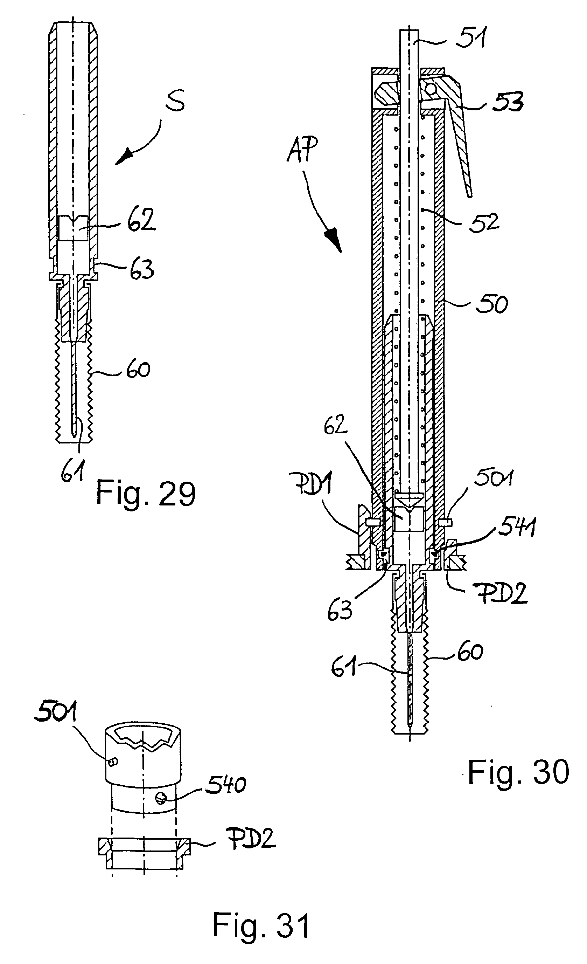

Accordingly, **Fig. 12** shows conveyor 2 in a position to load the spring of a spring loaded push rod 51, with upper containment 10 being shown dropped into opening 14. Conveyor 2 has been moved upwardly and, together with conveyor 2 a push rod 22 has been moved upwardly. In the "load spring" position, push rod 22 is arranged directly beneath pen docking station PD1, where the administration pen AP is docked to docking station DS for automatically making administration pen "ready for use". In **Fig. 13** only the lower end region of pen body 50 of administration pen AP is shown: The way how administration pen AP is docked to pen docking station PD1 as well as the overall structure of administration pen will be explained in more detail below. However, from Fig. 13 it can be seen that administration pen comprises a spring-loaded push rod 51 which can be moved upwardly through the upward movement of push rod 22 of docking station DS, thus tensioning the spring of spring-loaded push rod 51. Alternatively, it is also possible to tension the spring by metering the STH solution into syringe S only after an (empty) unused syringe S has been inserted into administration pen AP (see Fig. 30). The piston head 62 of syringe S (see Fig. 29) is then moved against the resilient forces of the spring of spring-loaded push rod 51.

It is to be mentioned, that the term "syringe" as used in connection with the instant invention does not denote a syringe in the conventional sense (stand-alone system). Rather, a syringe S is to be understood as a single use reservoir comprising a piston head 62 (see Fig. 29) and having an outlet with or without a needle 61. Only syringes S having a needle 61 are described in the following. The piston head 62 of syringe S can be actuated after having inserted the syringe S into administration pen AP (see Fig. 30) so as to move piston head 62 of syringe S in order to press the liquid STH solution through the outlet of syringe S, in the described embodiment through syringe needle 61.

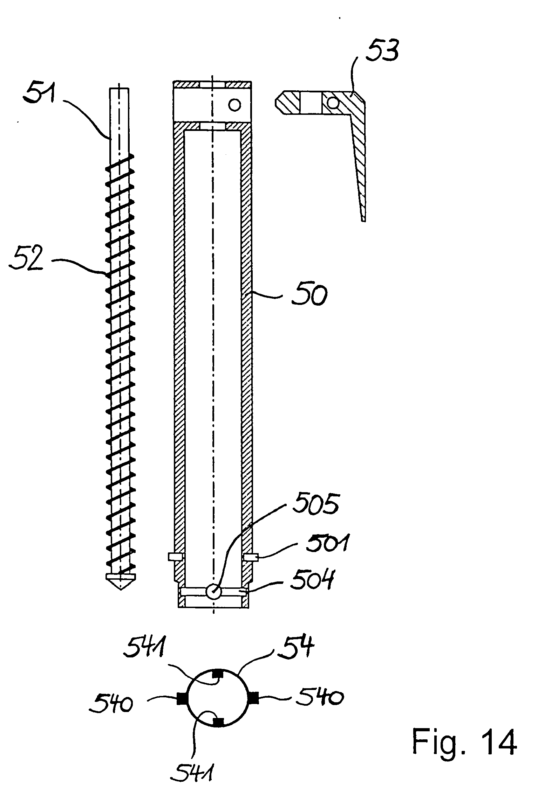

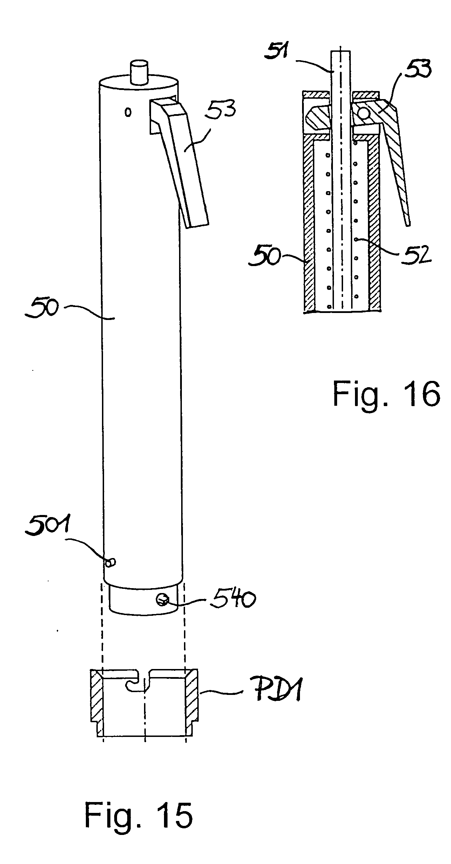

In **Fig. 14** the single parts of the administration pen are shown unassembled. The parts comprise a pen body 50, a push rod 51 provided with a spring 52, a release trigger 53 and a ring-shaped lock spring 54 having outwardly projecting protrusions 540 and inwardly projecting protrusions 541. Ring-shaped lock spring 54 serves for locking a syringe within administration pen AP and for later release of the syringe from the pen, as will be explained later. Ring-shaped lock spring 54 is mounted to pen body 50 by insertion into a respective groove 504 provided in the lower end region of pen body 50. Outwardly projecting protrusions 540 of ring-shaped lock spring 54 extend through holes 505 provided in the wall of pen body, while protrusions 541 project inwardly. Upon bajonet-locking administration pen AP to pen docking element PD1 with the aid of bajonet bolts 501 provided on pen body 50, protrusions 540 are not acted upon by the inner wall of pen docking element PD1, since the diameter of the inner wall of pen docking element PD1 is larger than the outer diameter of outwardly projecting protrusions 540 (see **Fig. 15**).

Release trigger 53 is mounted to pen body 50 by a pin such, that it is inclined. It is thus possible to move pushing rod 51 upwardly, but upon stopping the upward movement trigger 53 automatically clamps pushing rod 51 in the respective position (see **Fig. 16**).

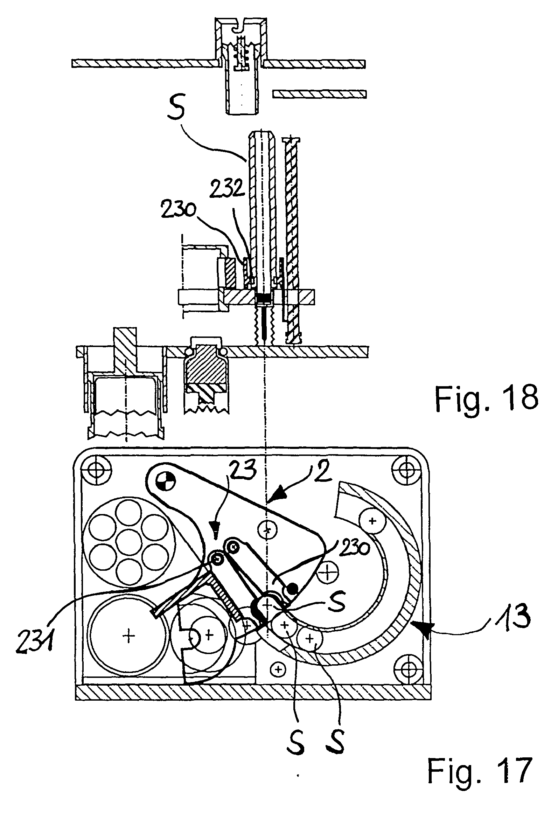

Now the operation of filling a metered dose of STH solution from lower containment 11 into a new syringe S will be explained. In a first step, conveyor 2 is moved to a pick-up position shown in **Fig. 17** and **Fig. 18**. Conveyor 2 is provided with a clamp 23 having to clamp arms 230 which can be pivoted about a pivot point 231 so as to open and close. In order to be able to grasp a new syringe S from the drum 13 (a spring load can be applied to the syringes S in the drum so as to bias them towards clamp 23) the clamp arms 230 are open. Clamp arms 230 are provided with projections 232 (see Fig. 18) which can engage into a groove provided in the outer wall of syringe S. This groove provided in the outer wall of the syringe can be seen best in Fig. 29 (see groove 63 in Fig. 29).

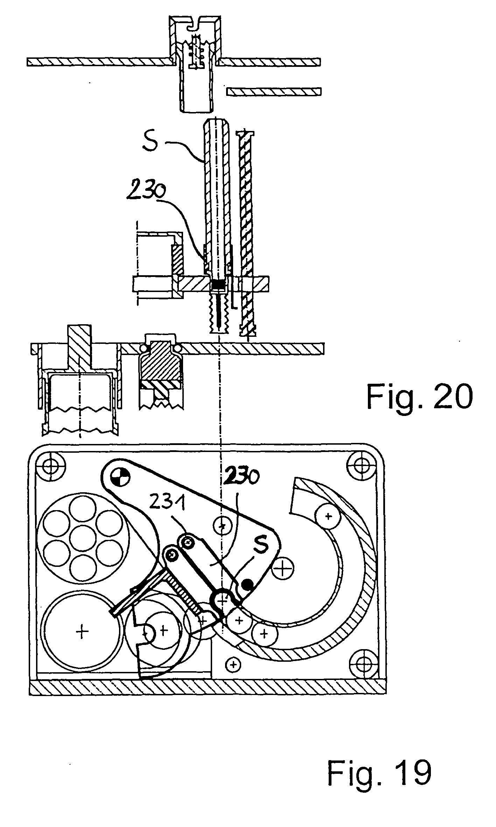

Clamp arms 230 can then be pivoted about pivot points 231 so as to close and engage into the groove provided in the outer wall of the syringe S. Conveyor 2 is still in the pick-up position. This is shown in **Fig. 19** and **Fig. 20**, respectively.

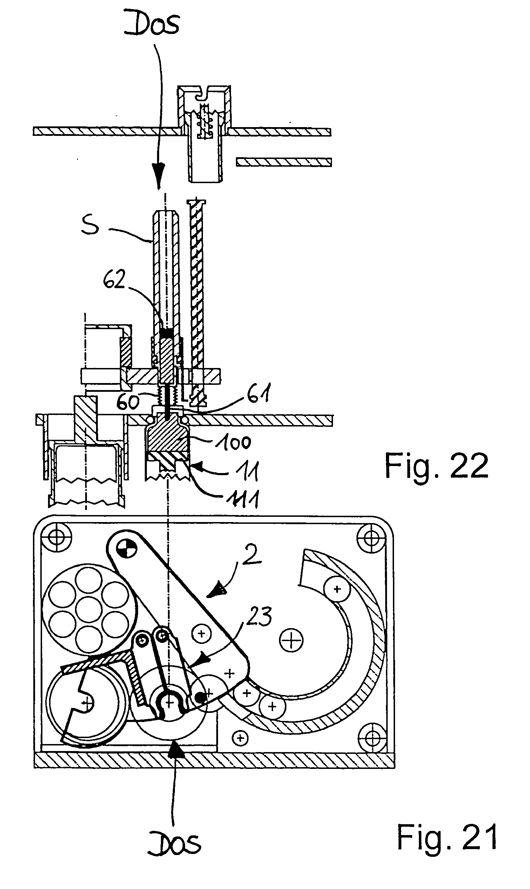

As can be seen in **Fig. 21** and **Fig. 22**, syringe S is then moved to the dosing station DOS, where conveyor 2 carrying syringe S is lowered. Syringe S is provided at its distal end with a bellows cover 60 fully covering syringe needle 61. This can also be seen best in Fig. 29. Bellows cover 60 not only provides a protection for syringe needle 61 but also hides the needle when the syringe has been transferred to administration pen. This may help people who are afraid of needles to self-administer the dose using the administration pen, since they do not see syringe needle 61. Turning back to Fig. 21 and Fig. 22, by lowering conveyor 2 the syringe needle 61 pierces through bellows cover 60 and establishes a communication with the STH-solution contained in lower containment 11. By moving piston 111 a predetermined distance, a metered amount of STH-solution 110 can be filled into syringe S, which also contains a small piston head 62. Piston head 62 can again be seen best in Fig. 29.

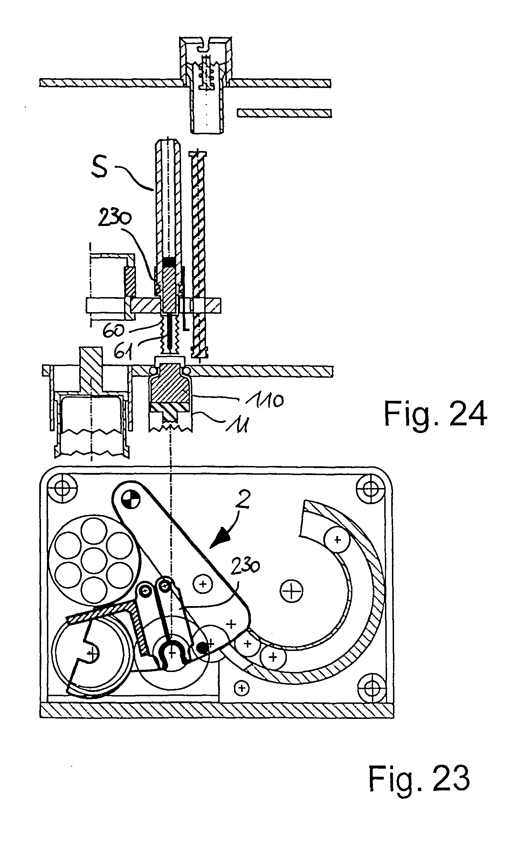

After having performed the dosing operation, conveyor 2 is lifted again thus terminating the communication between syringe needle 61 and the STH solution 100 contained in lower containment 11. Bellows cover 60 again covers syringe needle 61. This operation can be seen in **Fig. 23** and **Fig. 24**, respectively.

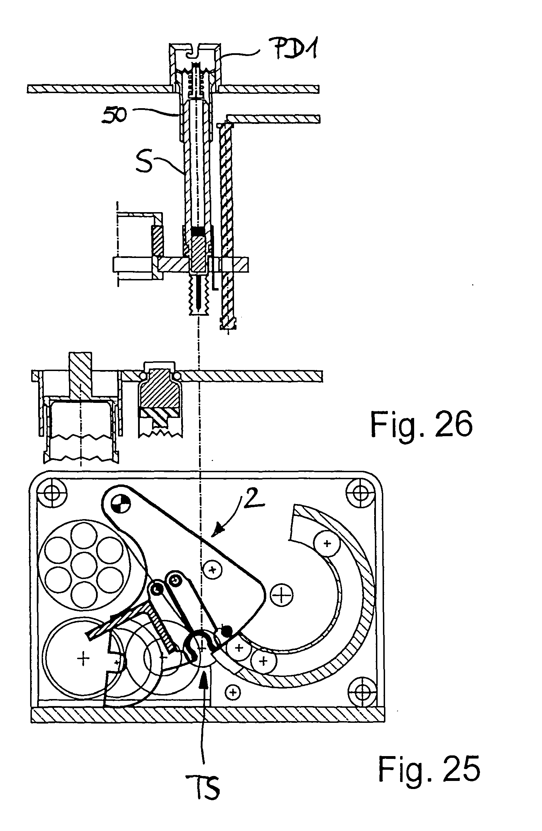

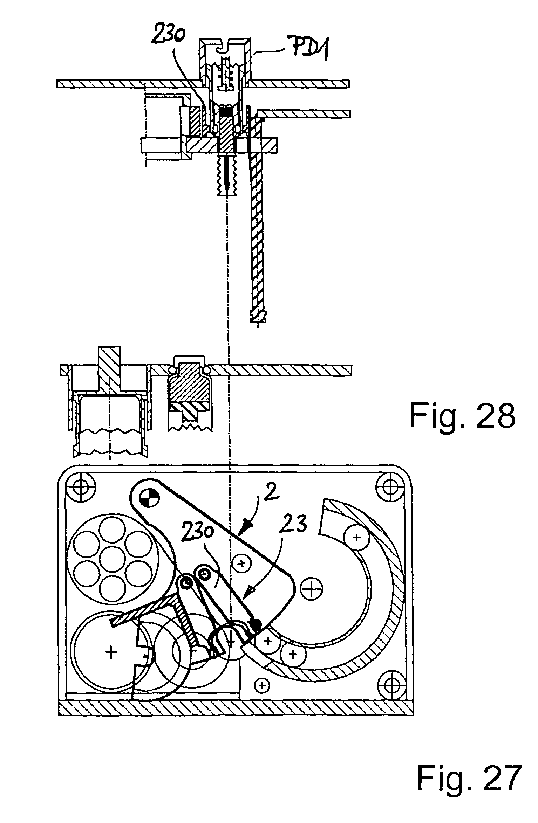

Conveyor 2 is now moved to the transfer station TS, in which syringe S is aligned with administration pen AP which is in engagement with pen docking station PD1 by a bajonet lock, as has already been explained. This can be seen in **Fig. 25** and **Fig. 26,** respectively. Conveyor 2 is then lifted thus inserting syringe S into the lower end of pen body 50 of administration pen until the inwardly protruding projections 541 of ring-shaped lock spring 54 (see Fig. 14) of administration pen AP engage into groove 63 provided on the outer wall of syringe S (see Fig. 29). Prior to insertion of syringe S into administration pen AP clamp arms 230 of clamp 23 are opened again widely, as this is shown in **Fig. 27** and **Fig. 28**, so as to allow the projections 541 of ring-shaped lock spring 54 to engage groove 63 of syringe S. In order to prevent syringe S from falling down, syringe S is supported at its lower end by conveyor 2, as can be seen in Fig. 28, while its upper end is guided in pen body 50 of administration pen. The engagement of ring-shaped lock spring 54 into the groove 63 of syringe S will be discussed again in more detail later.

As already mentioned, an enlarged view of syringe S is shown in **Fig. 29**. In Fig. 29 groove 63 provided on the outer wall of syringe S can be seen as well as a small piston head 62, which is moved upwardly upon the automatic filling of the metered dose of STH-solution into syringe S (see Fig. 22).

A fully loaded administration pen AP is shown in **Fig. 30** engaged with pen docking element PD1 by means of bajonet bolts 501. Also, pen docking station PD2 is indicated in Fig. 30 for illustration purposes. The tensioning of spring 52 by moving the push rod 51 upwards has been performed such that push rod 51 has moved upwards to a position that corresponds to the position of piston head 62 of syringe S. In order to correctly perform this operation, docking station DS is provided with an electronics (not shown) which needs to know the volume (dose) of STH-solution to be automatically metered into syringe S, e.g. the dose to be metered can be set by the physician. Once knowing the dose the electronics can determine the distance which the piston head 62 has to be moved and, accordingly, the electronics can determine the distance which the push rod 51 has to be moved upwards, so that the syringe can be properly accommodated within administration pen AP. The electronics also controls the other operations which are performed automatically (e.g. mixing, insertion of syringe S into administration pen), and may control complete use of STH solution of a multi-dose containment, storage of time and amount of STH solution administered, etc.. However, the electronics may also serve for additional purposes, such as providing an alarm system reminding the patient of administering the next dose, etc..

From Fig. 30 it can be seen that push rod 51 is clamped by release trigger 53. Syringe S is held in position through the engagement of projections 541 in groove 63 of syringe S. After removal of administration pen AP from pen docking station PD1, the pen is immediately "ready for administration". As can be seen, bellows cover 60 still hides syringe needle 61 making administration a little bit easier for patients which are afraid of syringe needles. Bellows cover 60 can be placed on the skin of the patient and by applying a slight pressure on pen body 50 in the direction towards the skin of the patient, syringe needle 61 pierces through bellows cover 60 into the skin of the patient, thus eliminating the need to see the needle. Once the needle has penetrated into the skin of the patient, a subcutaneous administration of the STH-solution can be performed by actuating release trigger 53, causing tensioned spring 52 to expand and consequently to move push rod 51 downwardly. Push rod 51 in turn moves piston head 62 of syringe 1 downwards thus pressing the liquid STH-solution out of syringe S through syringe needle 61. Of course, bellows cover 60 can also be removed prior to piercing syringe needle 61 into the skin of the patient.

Once administration of the STH-solution has been performed, the used syringe S can be transferred to syringe collector drum 12. For automatically performing this operation, administration pen AP can be moved to a collector station CS (see Fig. 5) comprising pen docking element PD2 (see Fig. 2 and Fig. 3). In **Fig. 31** the lower end of administration pen is shown above pen docking element PD2. It can be seen, that by insertion of the lower end of administration pen, the outwardly protruding projections 540 of ring-shaped lock spring 54 are compressed, thus deforming lock spring 54 such that inwardly protruding projections 541 (see Fig. 14) are slightly moved outwardly out of groove 63 of syringe S (see also Fig. 30) so as to allow syringe S to fall down out of administration pen AP and into collector drum 12. With the aid of the revolving disk RD (see Fig. 3), to which pen docking element PD2 is mounted, the respective opening of collector drum 12 can be selected into which the used syringe S is to be inserted.

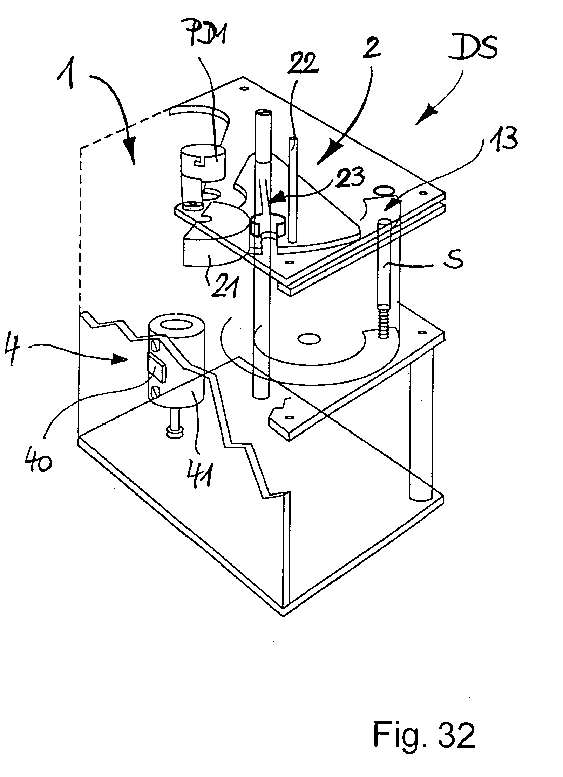

Finally, **Fig. 32** shows a three-dimensional representation of docking station DS schematically showing the positional arrangement of some elements of docking station DS. For example the cooling unit 4 comprising Peltier-element 40 and heat-conductive and heat-radiating casing 41 is schematically shown. Also, conveyor 2 is shown with carrier 21, push rod 22, and clamp 23, as well as drum 13 for providing new syringes. In addition, pen docking element PD1 for docking administration pen to docking station DS is shown, and a cut-out portion for insertion of cartridge 1 (see Fig. 2) to complete docking station DS. Also, it can be seen from Fig. 32 that there are different levels within docking station DS. These two levels are needed in the described embodiment of docking station DS, and this becomes immediately clear when looking at cartridge 1 as shown in Fig. 2 and Fig. 3.

While a specific embodiment of the administration device has been described with reference to the drawings the invention is not limited to this embodiment but rather is defined by the scope of the appended claims.

## Claims

1. Device for the administration of an essentially liquid compound or mixture of compounds (100,110), in particular for the administration of an essentially liquid medicament, comprising an administration pen (AP) for the administration of an individual metered dose of the liquid compound or mixture of compounds (100,110), **characterized in that** the device comprises a docking station (DS) to which the administration pen (AP) is to be docked (PD1), the docking station (DS) comprising a dosing station (DOS) for automatically metering the individual dose to be administered and a transfer station (TS) for automatically transferring the individual metered dose to the administration pen (AP).

2. Device according to claim 1, wherein the docking station (DS) further comprises a mixing station (MS) for automatically mixing a first (100) and a second (110) compound so as to form the essentially liquid mixture (100,110) of compounds to be metered.

3. Device according to any one of the preceding claims, wherein the docking station (DS) further comprises a cooling unit (4) for automatically storing the liquid compound or mixture of compounds (100,110) to be metered at a temperature within a predetermined temperature range.

4. Device according to any one of the preceding claims, wherein the docking station (DS) further comprises a syringe reservoir (13) for providing one or more unused syringes (S), and transport means (2,23) for automatically transporting an unused syringe (S) from the syringe reservoir (13) to the dosing station (DOS) for automatically filling the syringe (S) with the metered dose of the compound or mixture of compounds, and wherein the transfer station (TS) is adapted to automatically insert the filled syringe (S) into the administration pen (AP).

5. Device according to any one of the preceding claims, wherein the administration pen (AP) comprises an actuator means (52,53) to be actuated by the user, the actuator means comprising a tensioning means (52) and a release means (53), and wherein the docking station (DS) further comprises a loading means (22) for acting upon the tensioning means (52) such that the actuator means of the administration pen (AP) rests in a tensioned state.

6. Device according to claim 4 or claim 5, wherein the docking station (DS) further comprises a collector station (CS) for collecting used syringes (S) to which the administration pen can be docked (PD2) after administration, the collector station (CS) comprising means (PD2) for actuating on the administration pen (AP) upon docking of the administration pen in a manner such that the used syringe (S) is automatically transferred from the administration pen (AP) to the collector station (CS).

7. Device according to claim 6, wherein the administration pen (AP) comprises a lock means (54) for locking the syringe (S) within administration pen (AP) prior to insertion of the administration pen (AP) into the means (PD2) for actuating on the administration pen upon docking it to the docking station (DS), and wherein upon docking the administration pen to the docking station (DS) the lock means (54) of the administration pen (AP) are actuated by the means (PD2) for actuating on the administration pen so as to unlock the syringe (S) from the pen.

8. Device according to any one of the preceding claims, wherein the docking station (DS) or the administration pen (AP) comprises electronic means for the control and/or surveillance of the administration of the liquid compounds or mixture of compounds.

9. Cartridge (1) for insertion into a casing of a docking station (DS) of a device according to any one of the preceding claims, the cartridge (1) comprising a first containment (10) containing a plurality of doses of an essentially liquid compound or mixture of compounds, or containing a first component (100) of an essentially liquid mixture (100,110) of compounds to be administered in individual doses to a user, and the cartridge (1) further comprising a second containment (11) being empty or containing a second compound (110) to be mixed with the first compound (100) so as to form the essentially liquid mixture (100,110) of compounds to be administered.

10. Cartridge according to claim 8, further comprising a containment (12) for collecting used syringes (S).
